# EUROPEAN PATENT APPLICATION

(11) **EP 2 813 585 A1**
(43) Date of publication of application: **17.12.2014**
(21) Application number: 13003058.8
(22) Date of filing: 14.06.2013
(51) Int. Cl.: C22B 3/18, C22B 59/00

(54) **Process of isolating rare earth elements**

(71) Applicant: B.R.A.I.N. Biotechnology Research And Information Network AG, 64673 Zwingenberg (DE)
(72) Inventor: The designation of the inventor has not yet been filed
(74) Representative: Blodig, Wolfgang

(57) **Abstract**

A process of isolating a REE or a group of REE from a solution or dispersion containing said REE or said group of REEs comprising the following steps: (i) preparing a mixture comprising said solution or dispersion and biomass comprising at least one organism selected from any one of the following organism classes: eubacteria, archaea, algae, and fungi, whereby the at least one organism is capable of adsorbing or accumulating said REE or said group of REEs; (ii) incubating said mixture of step (i) for allowing the adsorption or accumulation of said REE or said group of REEs by said biomass; (iii) separating the biomass having adsorbed or accumulated REE(s) from the mixture of step (ii); and (iv) isolating said REE or said group of REEs from said biomass separated in step (iii).

## Description

### FIELD OF THE INVENTION

The invention relates to a process of isolating a rare earth element (REE) or a group of REEs from an aqueous solution or dispersion containing the REE or the group of REEs. Rare earth elements are isolated and concentrated from an aqueous solution by adsorption or accumulation by microorganisms and are subsequently released therefrom by separation procedures. The invention also provides a method of testing a sample microorganism for its ability to bind a rare earth element or of screening a plurality of microorganisms for the ability of members of said plurality to bind a rare earth element.

### BACKGROUND OF THE INVENTION

According to a recent classification the rare earth elements include the 17 elements scandium, lanthanium, cer, praseodym, neodym, promethium, samarium, europium (light rare earth elements, LREE) and yttrium, gadolinium, terbium, dysprosium, holmium, erbium thulium, ytterbium, lutetium (heavy rare earth elements, HREE). The demand for rare earth elements is growing steadily due to their importance, particularly in the field of high-tech electronics and displays. Further, a relevant share of the increasing demand is caused by so-called "green technologies" which aim at the reduction of energy consumption, development of renewable energy carriers and air pollution control. For example, rare earths are used in wind turbines, (hybrid) electric vehicles, automotive catalysts and energy-efficient lighting systems. Due to the increasing demand there is an urgent need for more efficient and sustainable mining processes even from low grade ores as well as efficient methods for recycling of rare earth metals, i.e. recycling them from waste material such electronic or metal scrap. To date, there has been no large scale recycling of REE from magnets, batteries, lighting and catalysts although the amounts of waste are substantial. The advantages of recycling REEs are amongst others the lack of radioactive impurities and economic independence from supply from primary sources. One reason for inadequate exploitation of these valuable resources, sometimes termed "urban mining", is that recycling processes for REEs are quite complex and energy-consuming, comprising physical and chemical treatment and generally the available know-how is still quite low.

### Conventional Beneficiation Processes

In most of the processes for REE beneficiation, the ore is mined in the deposit, broken down or milled and REE minerals are concentrated using physical properties such as density, magnetism and surface activities like electrostatic charge or flotation efficiency. The thus concentrated ore is then leached and the resulting REE-bearing solution is purified from undesired elements such as Fe, Ca, thorium and uranium. The process is described in e.g. Gupta, C.K. and Krishnamurthy, N. (2005): Extractive Metallurgy of Rare Earths., CRC Press (Florida, USA), and Castor, S.B. and Hedrick, J.B. (2006): Rare Earth Elements., In: Kogel, J.E., Trivedi, N.C., and Krukowski, S.T. (eds.): Industrial minerals and rocks: Commodities, markets, and uses., 7th edition, SME, page 769-792, as well as references therein. However, this leaching or 'cracking' stage depends on the type of minerals and other characteristics of the deposit. For instance at Mountain Pass, the mineral concentrate was calcined to drive off CO₂ and fluorine and leached with HCl to dissolve most of the trivalent REEs. Whereas the REE-bearing liquid was used for the separation of individual REEs, the residue (predominantly CeO₂) was sold. In contrast to that, the Bayan Obo REE mineral concentrate is baked with sulphuric acid at 300°C to 600°C and leached with water, taking REEs into solution and precipitating other elements as waste. REEs are then precipitated as double sulphates and converted to hydroxides (representing a chemical mixed rare earth concentrate), which are leached with HCl for the separation of individual REE. The varying composition and distribution of the individual REEs in the pregnant, REE-bearing solution or in the precipitated chemical mixed rare earth concentrate depends on the mineral deposit from which the ore originates. Following the leaching stage subsequent processing is required to separate individual REEs from each other. Separating individual REEs is a very difficult process due to their similar chemical properties. As a consequence the high value of REEs depends on their effective separation into high purity compounds. Cerium and europium can be separated by selective oxidation or reduction whilst other REEs can be separated in small amounts using fractional crystallisation or fractional precipitation. However, commercial separation generally is done using solvent extraction and, less common, ion exchange methods. Solvent extraction (SX), or liquid-liquid extraction, is a method used to separate compounds on the basis of their relative solubility's in two immiscible liquids, commonly the REE-containing aqueous solution and an organic solvent. On an industrial scale the solvent extraction is carried out in a group of mixer settlers, which allows repetitive fractionation during a continuously flowing process. Initially the process is relatively ineffective. When the process is repeated many times each REE can be separated from the others. However, the solvent extraction method is most appropriate for separating the LREEs, with the HREEs being more difficult to extract using this method. This is especially true if the used ore consists predominantly of the LREEs. Ion exchange is a process in which ions are exchanged between a solution and an insoluble (usually resinous) solid. The REEs from the solution displace the cations on the resin surface, whereas the aqueous waste containing the exchanged cations. Individual REEs are then separated using a complexing agent which has different affinities for the various REEs. The Ion exchange method produces highly pure REE in small quantities. However, it is a time consuming and thus expensive process. Consequently, only a small amount of HREEs are purified commercially on a small scale using ion exchange.

### Impact of conventional rare earth leaching on the environment and on health

The main environmental risks of conventional processes are due to tailings containing small-size particles, waste water and flotation chemicals. The tailings typically remain in the impoundment areas where they are continuously exposed to water e.g. from rain. Toxic substances are washed out, producing steady emissions to ground water. The composition of the polluting water is site-specific depending on the host minerals and the chemicals used for leaching and flotation. The tailings may contain radioactive substances, arsenic, fluorides, sulfides, acids and heavy metals. The refining of the rare earth concentrate is an energy-intensive and water consuming process and causes serious air emissions e.g. of SO₂, HCl, dust. Additionally, the solvent extraction method causes waste water, which is often extensively polluted by organic solvents like kerosene. Additionally, radioactive waste can arise, as the majority of rare earth deposits also contain thorium and/or uranium, thus radionuclides may pollute water and air. CO₂-emissions are also significant.

### Bioleaching

The possibility to use predominantly acidophilic, autotrophic iron-oxidising sulfur-oxidizing prokaryotes to recover precious and base metals from mineral ores and concentrates is known, e.g. from Rawlings, DE and Johnson, DB (The microbiology of biomining: development and optimization of mineral-oxidizing microbial consortia, Microbiology 2007, 153: 315-24). The development of this technology was inspired by the observation that certain bacteria, especially *Thiobacilli*, are able to solubilise heavy metal minerals by oxidizing Fe(II) to Fe(III) as well as sulfidic compounds to sulfate. This process is the major cause of natural weathering of sulfidic minerals.

By creating conditions that favour the growth of ore-decaying microorganisms, leaching of heavy metals from sulfidic minerals under aerobic conditions can be increased more than 100-fold compared to weathering without bacteria. However, degradation of minerals such as pyrite enclosing precious metal atoms or clusters can lead to the release of the trapped high-value compounds. While cheap *in situ* or dump/heap set-ups are generally used to bioleach base metals from low-grade rocks and minerals, more expensive (and more controlled) stirred-tank reactors are typically employed in the pretreatment of mineral ores for the recovery of metals. After the initial bacterial disintegration step, ores are subjected to a conventional chemical leaching process, hazarding the environmental and health problems mentioned above.

### Bioadsorption

A number of living microorganisms, but also nonviable, inactivated cells have the ability to bind metal ions. In the first case, metal binding can occur via adsorption to the cell surface or via active intracellular accumulation of metal ions. In the latter case of nonviable, inactivated cells - that is often referred to as *biosorption -* metal ion binding is believed to occur exclusively via surface adsorption. The biosorption capacity as a general characteristic of biomass results from the presence of chelating groups (e.g. carboxyl-, amide-, hydroxyl-, phosphate-, and thiol-groups) contributed by carbohydrates, lipids and proteins that are displayed on the cell surface. It has been described that amounts of metals of up to 50 % of the cell dry weight can be accumulated by biomass (Vieira and Volesky, 2000). United States Patent 1991/5055402 describes a process for removing metal ions from aqueous solution, using a matrix prepared from metal-binding microorganisms that have been heat-inactivated at temperatures of 300-500°C. However, specific binding mechanisms by organic surface structures are obviated by this procedure. EP 0673350 B1 describes the accumulation of metals, including some rare earth elements such as lanthanium and yttrium, by reacting phosphate ions generated by a microorganism and metals to polyphosphates. Accumulation of the metal-polyphosphates by the microorganism of the genus *Acmetobacter* makes the metals accessible to precipitation and depletion thus enabling purification of metal-polluted water. WO 1991/003424 describes a biomining procedure for leaching of gallium and germanium from ores using an admixture of bacteria, culture medium and crushed ore. However, no process has been described to date that could be used to recover REEs in significant amounts from ores or waste materials.

It is therefore an object of the present invention to provide a process of recovering, enriching or isolating REEs from source material, such as a mineral ore or a waste material containing REEs. It is another object of the invention to provide a process for modulating the composition of REEs in a solution, or isolating a particular REE, such as scandium or lutetium, from a solution. It is another object to provide organisms for these processes. It is a further object to provide a screening method for such organism.

### SUMMARY OF THE INVENTION

These objects are accomplished by:
(1) A process of isolating a rare earth element (REE) or a group of REEs from a solution or dispersion, comprising the following steps:
   (i) preparing a mixture comprising a solution or dispersion containing said REE or said group of REEs and biomass that comprises at least one organism selected from any one of the following organism classes: eubacteria, archaea, algae, and fungi, whereby the at least one organism is capable of adsorbing or accumulating said REE or said group of REEs;
   (ii) incubating said mixture of step (i) for allowing the adsorption or accumulation of said REE or said group of REEs by said biomass;
   (iii) separating the biomass having adsorbed or accumulated REE(s) from the mixture of step (ii); and
   (iv) isolating said REE or said group of REEs from said biomass separated in step (iii).
(2) The process according to (1), wherein said solution or dispersion is obtained by treating a source material of said REE or said group of REEs, such as a mineral ore, a mineral mining waste material, or an electronic or metal scrap, by an extraction or solubilising agent, such as an aqueous acid.
(3) The process according (2), wherein obtaining said solution or dispersion from said source material comprises pre-treating said source material with auto- or heterotrophic bacteria for bioleaching said REE or group of REEs from said source material before or concurrently to step (i).
(4) A process of isolating or enriching a rare earth element (REE) or a group of REEs from a particulate material, comprising the following steps:
   (i') preparing a mixture comprising a solution or dispersion prepared from said particulate material and biomass comprising at least one organism selected from any one of the following organism classes: eubacteria, archaea, algae, and fungi, whereby the at least one organism is capable of adsorbing or accumulating said REE or said group of REEs;
   (ii) incubating said mixture of step (i) for allowing adsorption or accumulation of said REE or said group of REEs by said biomass;
   (iii) separating the biomass having adsorbed or accumulated REE(s) from the mixture of step (ii); and
   (iv) isolating said REE or said group of REEs from said biomass separated in step (iii).
(5) The process according to (4), wherein said particulate material containing said REE or said group of REEs is a particulate mineral ore, a particulate mineral mining waste material or a particulate obtained from electronic scrap or scrap metal.
(6) The process according to (4) or (5), wherein said particulate material contains said REE(s) in the form of chemical compounds of the REE(s), such as carbonates, sulfates, oxides, phosphates or silicates.
(7) The process according to any one of (4) to (6), wherein said particulate material has an average particle size of at most 5 mm, preferably at most 1 mm, more preferably of at most 400 µm, and most preferably of at most 100 µm.
(8) The process according to any one of (4) to (7), wherein said particulate material is pre-treated with sulfide-oxidising bacteria for bioleaching said REE or group of REEs from said particulate material before or concurrently to step (i).
(9) The process according to (3) or (8), wherein said sulfide-oxidising bacteria are genetically-modified to express an S-layer on the surface of said bacteria.
(10) The process according to any one of (4) to (9), wherein said solution or dispersion is obtained by treating said particulate material by an extraction or solubilising agent such as an aqueous acid, optionally followed by adjusting the pH to a suitable pH for adsorption or accumulation of said REE or said group of REEs by said biomass.
(11) The process according to any one of items (4) to (10), wherein preparing said solution or dispersion from said particulate material comprises pre-treating said particulate material with autotrophic or heterotrophic bacteria for bioleaching said REE or group of REEs from said particulate material before or concurrently to step (i).
(12) The process according to any one of (1) to (11), wherein said solution or dispersion is aqueous.
(13) The process according to (12), wherein said aqueous solution or dispersion has a pH of from 0 to 5, preferably of from 0.5 to 3, more preferably of from 1.0 to 2.0; and/or said mixture of step (ii) has a pH of from 0 to 5, preferably of from 0.5 to 3, more preferably of from 1.0 to 2.0.
(14) The process according to any one of (1) to (13), wherein step (ii) comprises agitating said mixture for bringing said biomass in close contact with REE(s) present in said mixture.
(15) The process according to any one of (1) to (14), wherein said REE is selected from the group consisting of scandium, lanthanium, cer, praseodym, neodym, promethium, samarium, europium, yttrium, gadolinium, terbium, dysprosium, holmium, erbium, thulium, ytterbium, and lutetium; or said group of REEs comprises at least two REEs selected from the aforementioned list.
(16) The process according to any one of (1) to (14), wherein said REE is selected from the group consisting of lanthanium, cer, praseodym, neodym, promethium, samarium, and europium; or said group of REEs comprises at least two REEs selected from the aforementioned list.
(17) The process according to any one of items (1) to (14), wherein said REE is selected from the group consisting of terbium, dysprosium, holmium, erbium, thulium, ytterbium, lutetium, scandium, yttrium; or said group of REEs comprises at least two REEs selected from the aforementioned list.
(18) The process according to any one of (1) to (14), wherein said REE is selected from the group consisting of scandium, yttrium, gadolinium, terbium, dysprosium, holmium, erbium, thulium, ytterbium, lutetium; or said group of REEs comprises at least two REEs selected from the aforementioned list.
(19) The process according to any one of (1) to (14), wherein said REE is scandium.
(20) The process according to any one of (1) to (19), wherein the biomass is separated in step (iii) by one of the methods selected from centrifugation, filtration, flocculation and flotation.
(21) The process according to any one of (1) to (20), wherein step (iii) involves blowing of air into said mixture for accumulating biomass having bound REE at the surface of said mixture.
(22) The process according to any one of (1) to (21), wherein the incubation time of step (ii) is between 0.5 hours and 96 hours, preferably between 0.5 hours and 48 hours, and most preferably between 1 hour and 24 hours.
(23) The process according to any one of (1) to (22), wherein said organism is from genus *Cupriavidus, such as Cupriavidus metallidurans,* or said organism is *Citrobacter* sp.
(24) Use of biomass selected from the following organism classes: Eubacteria, Archaea, Algae, and Fungi for isolating a REE or group of REEs from a particulate material or a solution or dispersion containing said REE or said group of REEs. In one embodiment, the organism is from genus *Cupriavidus, such as Cupriavidus metallidurans,* or said organism is *Citrobacter* sp.
(25) A method of testing a sample microorganism for its ability to bind a rare earth element or of screening a plurality of microorganisms for the ability of members of said plurality to bind a rare earth element, comprising the following steps:
   (a) contacting a microorganism with a solution or dispersion containing a rare earth element (REE) or multiple REEs;
   (b) incubating the mixture of step (a) for a predetermined period of time;
   (c) separating microorganisms from the mixture obtained in step (b);
   (d) analysing the separated microorganism for bound REE.
(26) The method of (25), wherein said organism is selected from the following organism classes: Eubacteria, Archaea, Algae, and Fungi.
(27) The method of (25) or (26), wherein said solution or dispersion used in step (a) is an acidic aqueous solution or dispersion having a pH of from 0 to 5, preferably of from 0.5 to 3, more preferably of from 1.0 to 2.0: and/or wherein the mixture of step (b) has a pH of from 0 to 5, preferably of from 0.5 to 3, more preferably of from 1.0 to 2.0.
(28) The method according to any one of (25) to (27), wherein said predetermined period of time is between 0.5 hours and 96 hours, preferably between 0.5 hours and 48 hours, and most preferably between 1 hour and 24 hours.

The inventors have found that REEs can be isolated or enriched from a solution or dispersion, preferably an aqueous solution or dispersion, using biomass comprising organisms that can bind REEs. The invention provides a process for isolating REEs in a simple and cost-effective way. The biomass binds the REE or a group of REEs by cell components of the organisms. After separation of the biomass from unbound material, the REEs can be isolated from the biomass. The invention allows isolating REEs from sources that contain only low amounts of REEs, reducing the number of steps needed for REE separation compared to a conventional multistep process (raffination). Therefore, the processes of the invention provide an environmentally innocuous access to valuable REEs, that requires less energy and avoids pollution by transferring the mining procedure to a controlled containment. The present invention is a break-through in the sustainable exploitation of low-grade REE-sources, allowing the recovery of REEs in a simple process.

### BRIEF DESCRIPTION OF THE FIGURES

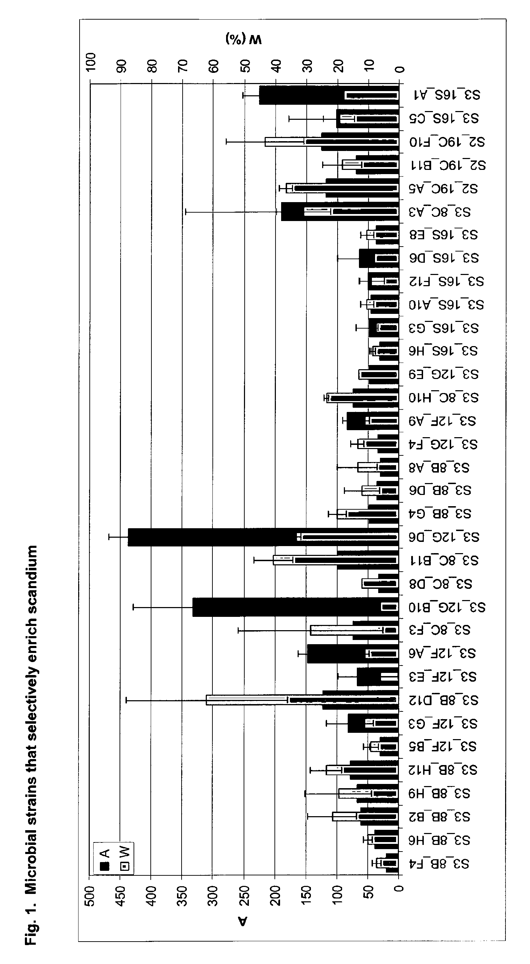

**Fig. 1****. Microbial strains that selectively enrich Scandium.** (A) Enrichment factors A (black bars) were calculated by A = [m_{BM}(Sc) * m_{L}(REE)] / [m_{BM}(REE) * m_{L}(Sc)]; m being the mass of scandium (Sc) or total REE (REE) in biomass (BM) and mineral leach (L), respectively. Recovery W (grey bars) was calculated by W = 100*m_{BM}(Sc)/m_{L}(Sc). Assays were carried out as described in Example 2 using a sulphuric acid leach of REE-containing bastnaesite. Designations on the x-axis refer to different microbial isolates.

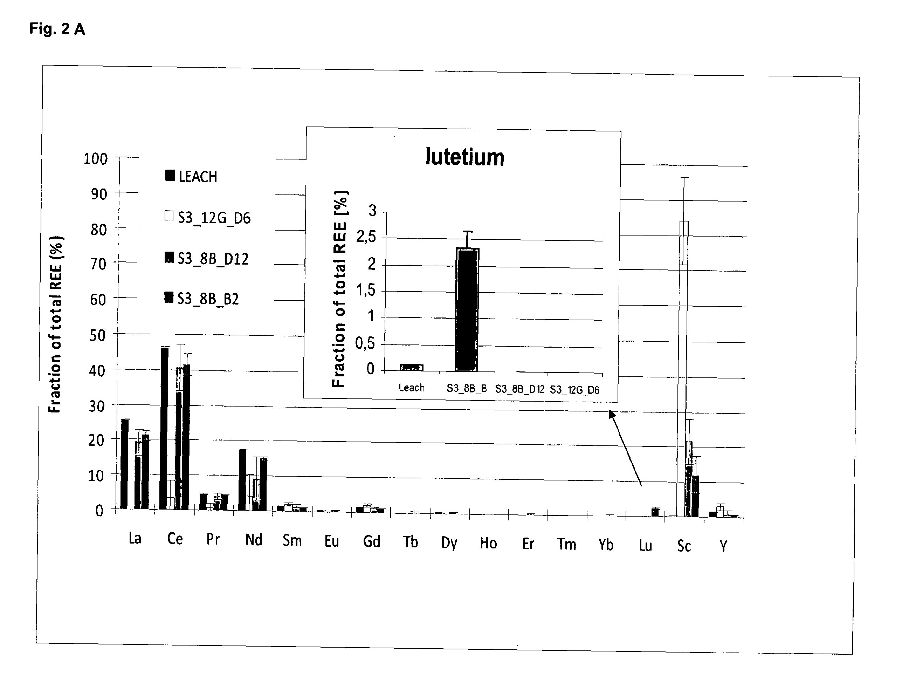

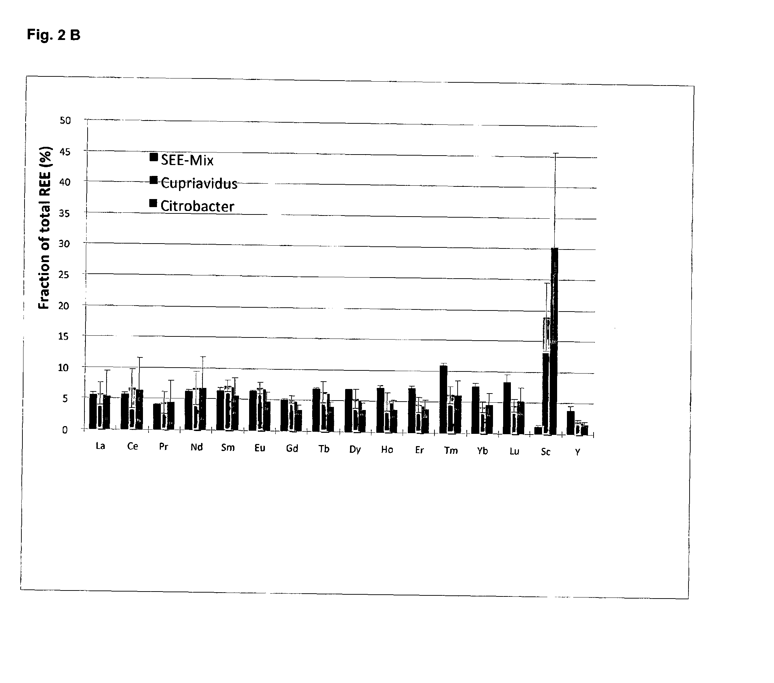

**Fig. 2****. Enrichment of specific REE from mineral leach by exemplary microorganisms.** REE composition of mineral leach as obtained by the treatment described in example 1 (black bars). Composition of REE extracted by the use of microbial biomass as described in Example 2. The y-Axis refers to fraction of total REE in percent by weight. **A.** Strain S3_12G_D6 strongly enriches scandium (A = 438). Strain S3_8B_B2 enriches scandium to a lower extent (A = 61), but also significantly accumulates the heavy REE Lutetium (A = 22). **B.** Composition of REE extracted by use of *Cupriavidus metallidurans* and *Citrobacter* sp. from an equimolar solution of 16 SEE.

### DETAILED DESCRIPTION OF THE INVENTION

The REE that may be isolated or enriched in the process of the invention is selected from lanthanium, cer, praseodym, neodym, promethium, samarium, europium (light rare earth elements, LREE) and scandium, yttrium, gadolinium, terbium, dysprosium, holmium, erbium, thulium, ytterbium, and lutetium (HREE). In one embodiment, the REE is selected from the group of light REEs, i.e. lanthanium, cer, praseodym, neodym, promethium, samarium, europium. In another embodiment, the REE is selected from the group of heavy REEs, i.e. scandium, yttrium, gadolinium, terbium, dysprosium, holmium, erbium thulium, ytterbium, lutetium. In a further embodiment, the REE is selected from the group consisting of terbium, dysprosium, holmium, erbium, thulium, ytterbium, lutetium, scandium, yttrium. Scandium is most preferred as the REE.

In the processes of the invention, groups of two or more REEs may be isolated or enriched. Such group may contain two or more REEs from any of the above-mentioned lists of REEs. A group of REEs may comprise two or more REEs from the light REEs or from the above list of heavy REEs.

The abbreviation "REE" stands for rare earth element. Multiple rare earth elements are abbreviated by "REEs". The term "REE(s)" covers the meaning of "REE" and "REEs". Herein, the term "REE" covers rare earth elements in elemental (metallic) form and chemical compounds comprising ionically or covalently bound REE ions or atoms. Dissolved ions of REEs e.g. in aqueous solution are also covered by the term "REEs". In the processes of the invention, the chemical form of the REE may change. The chemical form of the REE isolated in step (iv) may be different from the chemical form of the REEs in the source material of the REE. Frequently, the chemical form of the REE(s) will also be different in the solution of step (i) and in the form isolated in step (iv). The difference may be in terms of oxidation state and/or in terms of counter ions to cationic REE ions. It is possible that the solution or suspension used in the process of the invention contains REE(s) to be isolated in two or more different chemical states or compounds. Similarly, the REE(s) isolated in step (iv) may contain said metal in two or more different chemical states or compounds.

In natural resources such as in mineral ores, but also in mineral mining wastes, REEs usually occur as REE compounds such as complexes wherein the REE is present in oxidised form. As is known to the skilled person, the most prevalent oxidation states of REEs are the states +III and +IV. REEs in elemental (metallic) form are easily oxidised to form oxidised compounds, and generally react with mineral acids such as dilute sulfuric acid to form hydrogen and REE ions. Due to the stability of the oxidised state of the REEs, the REEs isolated in step (iv) of the present invention is generally a REE compound wherein the REE is present in oxidised form. Thus, the processes of the invention are, in one embodiment, processes of isolating compounds of REE(s). Known techniques may be used for preparing REE in elemental (metallic) form from REE compounds isolated in step (iv).

In the processes of the invention, the REEs generally bind to the biomass in the oxidised form of the REEs. Preferably, the REEs are contained in the mixture prepared in step (i) or (I') in dissolved form. This does not exclude that REE compounds finely dispersed in the mixture as small or colloidal particles are also adsorbed or bound by the biomass. Further, as soluble REE is preferably bound by the biomass in step (ii), insoluble forms of the REE may dissolve according to their equilibrium solubility under the conditions used.

The solution or dispersion containing the REE or the group of REEs is a liquid. The liquid phase is made up of a solvent. The solvent of the solution or dispersion may be water or an organic solvent or mixtures thereof. The organic solvent may be a polar solvent or a non-polar solvent, but is preferably a polar solvent. However, the solvent is preferably water or contains water, i.e. is aqueous. The aqueous solvent contains water and may additionally contain a polar organic solvent. Preferably, the aqueous solvent contains at least 50 % by mass water. The solvent is chosen such that a desired degree of solubility of the REE compounds to be isolated from the solution or dispersion is obtained. Since the solubility of REE compounds in solvents such as water and other aqueous solutions is generally higher in the acidic range, the solvent may contain an acid. Inorganic (mineral) acids such as sulfuric acid or hydrochloric acid are preferred, but organic acids may also be used alone or together with inorganic acids. In aqueous solutions or dispersions, the pH may be from 0 to 5, preferably from 0.5 to 3, more preferably from 1.0 to 2.0. Upon addition of the biomass in step (i), the pH or acidity may change. Accordingly, the pH or acid content may be readjusted after addition of the biomass in order to maintain the desired acidity or pH for step (ii) of the process. The mixture of step (ii) may have a pH of from 0 to 5, preferably of from 0.5 to 3, more preferably of from 1.0 to 2.0.

The solution or dispersion containing said REE or group of REEs to be used in step (i) may be obtained or prepared in different ways that depend to the source material of the REE(s). In one embodiment, the REE(s) are already present in the form of a solution or dispersion in the source material, such as in liquid tailings from mining industry or liquids that form from rain falling on piles of solid mining waste material and washing out REE(s) from the waste material. In such cases, the solutions or dispersions may be used for step (i) as they are, optionally after adjustment of conditions such as concentration and/or acidity.

In another embodiment, the source material of the REE(s) is metal or electronic scrap that may contain the REE(s) in metallic elemental form or chemically bound or both. Metallic REE(s) may be separated and enriched mechanically. Metallic REE(s) to be isolated using the process of the invention are generally transformed chemically to soluble form, e.g. by treatment with aqueous organic or mineral acids, such as sulfuric acid or hydrochloric acid to form the respective REE salts of such acids, such as sulfates or chlorides, respectively, in aqueous solution or dispersion. Also REE compounds that are insoluble or poorly soluble in water at neutral pH may solubilised by such acids. The solutions or dispersions of the REEs used in step (i) may be acidic as described above, since the solubility of REE salts is generally higher in acidic solutions. The pH and other conditions may be adjusted as required for step (ii).

In other embodiments, the source material of the REE(s) are mineral ores or solid mine waste material obtained from processes of producing other desired components from ore. In these cases, the source material is generally first ground to fine particulate material for improving accessibility and leachability of REE(s) contained therein. Examples of mineral ores containing REE(s) are bastnaesite, thortveitite, monazite, loparite, gadolinite, euxerite, eschynite, allanite, apatite, britholite, brockite, cerite, fluorcerite, fluorite, parisite, stillwellite, synchisite, titanite, xenotime, zircon, zirconolite, etc. The mineral ores generally contain the REE(s) in the form of chemical compounds such as carbonates, sulfates, oxides, phosphates or silicates. To ensure efficient solubilisation and extraction of the REEs from the ores or mining waste material, these should be finely ground to form particulate material. The particulate material may have an average particle size of at most 5 mm. Alternatively, the particulate material may have an average particle size of at most 1 mm, of at most 400 µm, or of at most 100 µm, or of at most 50 µm, or of at most 30 µm.

For preparing the solution or dispersion for step (i) from mineral ores or mining waste material as source material, the preferably comminuted particulate material may be extracted (leached) with suitable solvents such as with organic solvents, bases or organic or inorganic acids, preferably with inorganic acids such as with dilute sulfuric acid or hydrochloric acid, whereby the REE compounds are dissolved in the solvent. The acid concentration in the dilute organic or mineral acid is not particularly limited, but should be at least 2% by weight for ensuring sufficient efficiency. The concentration may be from 5 to 20 % by weight or from 7 to 15 % by weight. Alternatively, other methods suitable to facilitate REE release and solubilisation may be employed, such as oxidative or heterotrophic bioleaching or incubation with microorganisms that produce corrosive metabolites. After the extraction, solid material may be removed e.g. by filtration or sedimentation. Furthermore, bioleaching procedures may be used to release the REE(s) of the invention from the particulate material. Autotrophic or heterotrophic bacteria may be used for pre-treating the source material or the particulate material for bioleaching. For instance, if said particulate material is a sulfidic ore such as pyrite, sulfide-oxidizing bacteria such as Thiobacilli may be used for at least partially degrading the sulfidic mineral. Such bioleaching is described by Rawlings, DE and Johnson, DB (The microbiology of biomining: development and optimization of mineral-oxidizing microbial consortia, Microbiology 2007, 153: 315-24). Bioleaching may be carried out before or after solvent extraction. If it is carried out after solvent extraction such as with strong acid or bases, a step of bringing the acid or base content of the particulate material to a level suitable for the bacteria used for bioleaching may be necessary. In step (i'), bioleaching may be done concurrently with step (i') and subsequent step (ii) by adding the bacteria for bioleaching to the biomass of step (i'). In one embodiment, the sulfide-oxidising bacteria are genetically-modified to express an S-layer on the surface of said bacteria.

In step (i) of the process of the invention, a mixture is prepared from said solution or dispersion containing the REE or group of REEs to be isolated and said biomass. In step (i'), a mixture is prepared from the biomass and a solution or dispersion obtained from the particulate matter, whereby the particulate matter may still be present in the mixture. If the particulate matter is still present, extraction and/or bioleaching may take place or continue to take place concomitantly with binding of the REE(s) by the biomass in step (i') and subsequent step (ii). If the acidity of the solution from a previous leaching step is too high, the pH may be increased by addition of bases to reach a pH that is compatible with the biomass used in steps (i), (i') and (ii). Preferred pH ranges for these steps are given below.

Steps (i) and (i') and subsequent step (ii) may be conducted in closed reactors that preferably contain an agitation system for agitating the mixture. The reactor may be a stirred-tank reactor and may be operated in a batch or continuous-flow mode. The reactor is preferably equipped with devices for measuring and controlling process parameters such as temperature, pH, etc.

The biomass used in step (i) or (i') comprises organisms, preferably microorganisms, selected from Eubacteria, Archaea, Algae, and Fungi. Among these, Eubacteria, Archaea and Algae are preferred, Eubacteria and Archaea are more preferred and Eubacteria are most preferred. The microorganisms used may naturally or by state-of-the-art genetic engineering have the potential to bind REEs. Generally, the REEs are bound in the oxidised form of the REEs. This property is exploited to adsorb or accumulate REEs that are present in the mixtures of steps (i), (i') and (ii). The organism to be used depends on the type of REE or group of REEs to be isolated. Other criteria for the choice of the biomass may be the chemical state of the REE present in the mixtures. Suitable organisms for a given REE or REE compound can be identified by screening large strain collections using the procedure described below and in Example 1. Screening may be done following the procedure of Example 1 or Example 2. In the research that led to the invention, microorganisms were assayed for their ability to grow in the presence of REEs and, in a secondary screening, to bind and/or accumulate REEs. An alternative to screening a broad diversity of organisms is the pre-selection of microbes that belong to phylogenetic groups that have turned out to have high metal-binding potential. The biomass may bind the REEs by adsorption to the cell surface or cell wall components, or via active intracellular accumulation of ions of the REEs. Microorganisms carrying homologous or heterologous metal-binding or modifying structures such as S-layers, polysaccharides, metal-reducing enzymes, metallothioneines, phytochelatins or surface-bound natural metallophores are suitable organisms for the present invention.

Microorganisms, particularly eubacteria and archaea, fungi and algae that have the required REE-binding affinity and specificity can be isolated from environmental sources, using known microbiological techniques. Environmental sources (habitats) that contain organisms suitable for the present invention are, however not exclusively, sediments and waters exposed to heavy metal or radionuclide contamination, such as acid mine drainages, electroplating effluents, mining waste piles, industrial effluents, and waste water treatment plants. Microorganisms viable and competitive in these environments often have adopted strategies to efficiently bind and immobilize heavy metals either on their surface or in their interior in order to reduce their toxicity. Typically, cell envelopes of microorganisms exhibit negative charges, enabling the adsorption of cationic metals. The main functional groups that contribute to this negative charge are phosphate moieties and carboxylic groups.

Organisms that do not naturally have metal-binding components can be provided with components allowing binding of the REE of the invention by genetic engineering. For instance, DNA fragments encoding genes or pathways that lead to the formation of metal-binding or metal-immobilizing structures can be introduced into wild-type strains, using techniques known to those skilled in the art. The present invention makes use of microorganisms that naturally - or by genetic engineering - have the potential to bind REEs.

Examples of natural or genetically-engineered components of organisms that may be used for binding the metal of the invention are the following.

**Metallothionines.** These metal-chelating polypeptides have been identified in many groups of organisms, including mammals, nematodes, fungi, and bacteria. Metallothioneines are characterized by an extremely high cysteine content of up to 33% arranged in (Cys-X-X-Cys) or (Cys-X-Cys) clusters and the absence of aromatic and hydrophobic amino acids.

**Phytochelatins.** Phytochelatins typically occur in plants and algae and are short, non-translationally synthesized polypeptides with variously repeating gamma-glutamylcysteine units (γGlu-Cys)ₙGly (n = 2-11). Synthetic phytochelatins [(Glu-Cys)nGly] have the advantage that they can be synthesized by the ribosomal machinery and that in some cases they bind metals even more effectively than the natural phytochelatins.

**S-layers.** Paracrystalline proteinaceous surface layers (S-layers) occur as surface structures in almost all major phylogenetic groups of bacteria and in almost all archaea (Sara and Sleytr 2000). The proteins (40-200 kDa) are secreted and subsequently self-assemble on the bacterial membrane, forming a very regular nano-porous structure (30-70% porosity). S-layer proteins constitute up to 20% of all cellular proteins. Due to their high content in hydrophobic amino acids, S-layer lattices in general render prokaryotic cell walls less hydrophilic, which can lead to increased foaming during cultivation. Immobilization of metals on S-layer templates has been used in nanotechnology to synthesize metallic nanoclusters of the precious metals Au (Dieluweit, Pum et al. 1998; Györvary, Schroedter et al. 2004) and Pt and Pd (Wahl, Mertig et al. 2001).

**Polysaccharides.** Some microorganisms produce biopolymers, e.g. polysaccharides that are able to bind 0.1 mg to 1.4 g metal/g isolated polymer, depending on the microorganism under investigation and the specific metal (Gutnick and Bach 2000). Binding generally occurs via electrostatic interactions between negatively charged groups in the biopolymer and the positively charged metal or via chelation of the metal by hydroxyl groups.

Examples of suitable microorganisms to be used in the present inventiono are microorganisms from genus *Cupriavidus,* such as *Cupriavidus metallidurans.* An example of *Cupriavidus metallidurans* is DSMZ Type strain 2839. Another example is *Citrobacter* sp. These microorganisms are preferably used in the methods and uses of the invention for isolating or enriching scandium.

In the process of the invention, it is possible to combine two or more microorganisms in the biomass. For example, different microorganisms each preferentially binding a particular REE (or groups thereof) may be combined for increasing the variety of REEs that may be isolated in the process. Depending on the composition of the particulate material, two or more microorganisms can be combined in said biomass to recover different chemical forms of REEs or different REEs in parallel.

In one embodiment, microorganisms for the processes of the invention are eubacteria and archaea. Microorganisms of the genera of *Pseudomonas, Cupriavidus* or *Bacillus* are preferred. A preferred species from genus *Cupriavidus* is *Cupriavidus metallidurans* such as DSMZ type strain 2839.

In another embodiment, the biomass used in the invention is or contains an organism belonging to eubacteria or archaea for adsorbing or accumulating scandium as the REE. For this purpose, microorganisms of the genera of *Pseudomonas, Cupriavidus* or *Bacillus* may be used. A preferred species from genus *Cupriavidus* is *Cupriavidus metallidurans* such as DSMZ type strain 2839 for scandium isolation.

The biomass used in the invention may be viable or dead. Native cells as obtained by cultivation in growth media (wet biomass) as well as dry biomass, e.g. obtained by freeze-drying or by drying at elevated temperatures can be used. Temperatures applied during drying should not exceed 100°C in order to prevent thermal degradation of cell components that are involved in specific REE adsorption. Preferably, however, the biomass used in step (i) and (i') is viable, i.e. contains viable cells of the organisms used. The conditions in the mixtures of steps (i), (i') and (ii) may be such that the organisms in the mixture remain viable to a large extent and may even grow further in the step (ii). In an embodiment where the organisms of the biomass should stay viable in step (ii), conditions have to support viability. For this purpose, the mixtures may contain nutrients required for the biomass. Further, air by be blown into the mixtures for providing oxygen to the biomass. Suitable growth conditions and nutrient requirements for the organisms can be obtained from the general prior art on microbiology. Suitable growth conditions are also provided by collections of microorganisms such as the American Type Culture Collection (ATCC) or the German Collection of Microorganisms and Cell Cultures (Deutsche Sammlung von Mikroorganismen und Zellkulturen GmbH, DSMZ) where members of the classes of microorganisms mentioned above can be obtained from.

In another embodiment, the biomass added in step (i) or (i') is viable, but is allowed to fully or partly die in the course of the process. Generally, the biomass may at least partly die due to unfavourable conditions in the mixture such as acidic pH.

In step (ii) of the processes of the invention, the mixture of step (i) or (i') is incubated for allowing binding such as adsorption or accumulation, of said REE or said group of REEs by said biomass. Step (ii) should be conducted for a time period sufficient to allow the biomass to adsorb and/or accumulate the REE(s) from the solution. The incubation time may be chosen such that no or little more REE(s) are absorbed or accumulated by the biomass at the end of the incubation step. The incubation time depends on the rate of binding. Generally, the incubation time is between 0.5 hours and 96 hours, preferably between 0.5 hours and 48 hours, and more preferably between 1 hour and 24 hours. The temperature of incubation depends mostly on the type of biomass used and the REE that are to be recovered. Step (ii) may comprise agitating said mixture for bringing said biomass in close contact with particles of said particulate material. As indicated above with respect to step (i), the reactor in which step (ii) is carried out may be equipped with devices for measuring and controlling process parameters such as temperature, pH, etc.

In step (iii), said biomass having bound REE(s) is separated from the mixture of step (ii). Known methods may be used for the separation. For example, the metal-loaded biomass may be separated from the solution by centrifugation or filtration. Alternatively, flocculation or flotation may be used. The separated biomass may, depending on the subsequent step, be dried for facilitating storage and/or transport and/or metal separation in step (iv).

In step (iv), the metal bound to the biomass is isolated from said biomass. The metal may for example be desorbed from the biomass in a liquid phase using acidic or basic conditions, or elution with chemicals such as chelating agents that can form soluble complexes with the REEs. Alternatively, the biomass may be combusted to destroy and remove organic matter of said biomass, whereby the REE can be isolated from ashes or fumes. Further, mechanical means may be used for separating the REEs from the biomass, such as sonication. The REE(s) may be purified from the residues, ashes or fumes of the biomass. Preferably, the isolation method allows recycling of the biomass for use in further REE-extraction processes.

In one embodiment, the mixture from step (ii) may be poured in step (iii) into chromatography columns that holds back the biomass but allows removal of excess liquid from the column. In this embodiment, step (iv) may be performed by eluting REEs from the column using a liquid medium as eluent that weakens the binding of the REEs to the biomass such as complexing agents. In this way, the REEs, notably soluble compounds thereof, may be obtained in concentrated eluent.

The processes of the invention may be combined with process steps used for isolating specific REE(s) from metal solutions known from prior art. For example, where a group of REEs was isolated in step (iv), individual REEs or compounds thereof may be isolated. If desired, REEs in elemental form may be generated from REE compounds by known reduction methods.

The invention also provides a method of testing a sample microorganism for its ability to bind a REE, and a method of screening a plurality of microorganisms for the ability of members of said plurality to bind a rare earth element, comprising the following steps:
(a) contacting a microorganism with a solution or dispersion containing a rare earth element or multiple REEs;
(b) incubating the mixture of step (a) for a predetermined period of time;
(c) separating microorganisms from the mixture obtained in step (b);
(d) analysing the separated microorganism for bound REE.

In step (a), a microorganism is contacted with a solution or dispersion containing a REE or multiple REEs. Similarly as described above, the microorganism may be selected from the following organism classes: Eubacteria, Archaea, Algae, and Fungi. The REE may be solution, preferably an aqueous solution, of the REE for which the binding ability of the microorganism is to be tested. Preferably, the solution contains two or more REEs, since this allows testing the binding ability of the microorganism to multiple REEs in parallel if the separated microorganism is analysed in step (d) for the multiple REEs.

For increased solubility of the REE in the solution, the solution may contain an acid such as those mentioned above. The solution or dispersion used in step (a) may be an acidic aqueous solution or dispersion. The pH of the solution or dispersion may be from 0 to 5, preferably of from 0.5 to 3, more preferably of from 1.0 to 2.0. The pH of the mixture of step (ii) may be from 0 to 5, preferably from 0.5 to 3, more preferably from 1.0 to 2.0.

In incubation step (b) and the separation step (c) may be carried out as described above for steps (ii) and (iii) of the process of isolating a REE.

In step (d), the separated microorganism is analysed for bound REE. This may involve extraction of metal compounds from the separated microorganism e.g. using a mixture of concentrated nitric acid and hydrochloric acid, preferably in combination with heating. The extraction acid may then be diluted with pure water and subjected to any generally known method for analysing REEs such as atom absorption spectroscopy or inductively coupled plasma mass spectroscopy (ICP-MS). These methods may be used for quantitative analysis of REEs in the separated microorganism. If multiple REEs are detected, their relative abundance may be compared with the relative abundance of multiple REEs in the starting REE solution. Thus, enrichment of one or more particular REEs compared to others can also be detected. In this way, a suitable or optimal microorganism for a particular purpose may be found.

### EXAMPLES

### Example 1: Preparation of mineral leach

Bastnaesit ore was leached using 10%-H₂SO₄ in a 1:5 (w/v) ratio of ore and acid. A 50-g sample of ore was incubated under continuous stirring with 250 ml of 10%-H₂SO₄ for 3 days at room temperature. The leach solution was centrifuged to remove non-dissolved particles and the supernatant was used as so-called "mineral leach" for further experiments. Before each experiment, the pH of mineral leach was adjusted to 1.3 by the addition of 10N NaOH.

### Example 2: Screening for microorganisms that enrich scandium from mineral leach

Microbial strains that have the potential for adsorbing/accumulating REE were selected by their ability to grow on solid media (Luria Bertani (LB) medium: 10 g/I tryptone and 5 g/I yeast extract, with 1.5 g/I agar) containing amounts of 1 to 5 mM REE (single elements or mixtures). REE-resistant microorganisms were screened for their ability to enrich scandium (Sc) from mineral leach. To this end, microbial strains were cultivated in LB medium (without agar) at the 50-ml scale according to standard microbiological techniques. Cells were collected in the stationary phase by centrifugation. Amounts of 20 OD units were incubated for 1 h at room temperature (25°C) with 1 ml of mineral leach. After incubation, cells were collected by centrifugation and washed once with 100 µl 10%-H₂SO₄. As a control for spontaneous (chemical) precipitation of REE, 1-ml aliquots of mineral leach without biomass were used. Cell pellets and precipitates were extracted by nitrohydrochloric acid for 2 h at 100°C using a Digi-Prep sample preparation device (S-Prep, Überlingen, Germany). After dilution in ultrapure water, REE contents of the cell pellets were determined by ICP-MS (Agilent, 7700 ICP-MS).

### Results

A number of 36 microbial strains (hit candidates) were detected that selectively bind Sc in their biomass. Enrichment factors for Sc compared to the other REE of up to 438 were observed (Fig. 1). Analysis of partial 16S rDNA sequences suggested that many hit candidates originate from the groups of *Pseudomonas* and *Bacillus.*

### Example 3: Scandium recovery from mineral leach by microbial biomass

Strains S3_8B_D12 and S3_8B_B2 were used to determine Sc recovery in an experimental set-up as described in Example 2. As shown in Fig. 2 A, S3_8B_D12 was able to enrich Sc by a factor of 438 compared to the original mineral leach (black bars, REE = w/w), leading to a REE mixture that contains more than 80% of the target element. By the applied single-step extraction, 33% of the present Sc could be recovered. With strain S3_8B_B2 only an enrichment factor of 61 could be achieved for Sc (W = 22%). On the other hand, however, also Lutetium - a very underrepresented heavy REE - could be enriched by a factor 22, leading to a recovery of 8% of the present material.

We used DSMZ Type strain 2839 (*Cupriavidus metallidurans*) and *Citrobacter* sp. to recover REE from an equimolar solution (1 mM each, in 10%-H₂SO₄; pH adjusted to 2.2 by addition of 1N-NaOH) of all 16 stable REE (Fig. 2B, black bars = SEE-mix, percent (w/w)). 20 OD units of cells originating from a stationary phase (overnight) culture prepared in LB medium were incubated for 1 h with 1 ml of said REE solution. After incubation, cells were collected by centrifugation and washed once with 100 µl 10%-H₂SO₄. ICP-MS analysis was carried out as described above.

## Claims

1. A process of isolating or enriching a rare earth element (REE) or a group of REEs from a solution or dispersion containing said REE or said group of REEs, comprising the following steps:
(i) preparing a mixture comprising said solution or dispersion and biomass comprising at least one organism selected from any one of the following organism classes: eubacteria, archaea, algae, and fungi, whereby the at least one organism is capable of adsorbing or accumulating said REE or said group of REEs;
(ii) incubating said mixture of step (i) for allowing the adsorption or accumulation of said REE or said group of REEs by said biomass;
(iii) separating the biomass having adsorbed or accumulated REE(s) from the mixture of step (ii); and
(iv) isolating said REE or said group of REEs from said biomass separated in step (iii).

2. The process according to claim 1, wherein said solution or dispersion is obtained by treating a source material of said REE or said group of REEs, such as a mineral ore, a mineral mining waste material, or an electronic or metal scrap, by an extraction or solubilising agent, such as an aqueous acid.

3. A process of isolating or enriching a rare earth element (REE) or a group of REEs from a particulate material, comprising the following steps:
(i') preparing a mixture comprising a solution or dispersion prepared from said particulate material and biomass comprising at least one organism selected from any one of the following organism classes: eubacteria, archaea, algae, and fungi, whereby the at least one organism is capable of adsorbing or accumulating said REE or said group of REEs;
(ii) incubating said mixture of step (i) for allowing adsorption or accumulation of said REE or said group of REEs by said biomass;
(iii) separating the biomass having adsorbed or accumulated REE(s) from the mixture of step (ii); and
(iv) isolating said REE or said group of REEs from said biomass separated in step (iii).

4. The process according to claim 3, wherein said particulate material containing said REE or said group of REEs is a particulate mineral ore, a particulate mineral mining waste material or a particulate obtained from electronic scrap or scrap metal.

5. The process according to claim 3 or 4, wherein said particulate material contains said REE(s) in the form of chemical compounds of the REE(s), such as carbonates, sulfates, oxides, phosphates or silicates.

6. The process according to any one of claims 3 to 5, wherein said particulate material has an average particle size of at most 5 mm, preferably at most 1 mm, more preferably of at most 400 µm, and most preferably of at most 100 µm.

7. The process according to any one of claims 3 to 6, wherein said solution or dispersion is obtained by treating said particulate material by an extraction or solubilising agent such as an aqueous acid, optionally followed by adjusting the pH to a suitable pH for adsorption or accumulation of said REE or said group of REEs by said biomass.

8. The process according to any one of claims 3 to 7, wherein preparing said solution or dispersion from said particulate material comprises pre-treating said particulate material with autotrophic or heterotrophic bacteria for bioleaching said REE or group of REEs from said particulate material before or concurrently to step (i).

9. The process according to any one of claims 1 to 8, wherein said solution or dispersion is aqueous.

10. The process according to claim 9, wherein said aqueous solution or dispersion has a pH of from 0 to 5, preferably of from 0.5 to 3, more preferably of from 1.0 to 2.0;
and/or said mixture of step (ii) has a pH of from 0 to 5, preferably of from 0.5 to 3, more preferably of from 1.0 to 2.0.

11. The process according to any one of claims 1 to 10, wherein said REE is selected from the group consisting of scandium, lanthanium, cer, praseodym, neodym, promethium, samarium, europium, yttrium, gadolinium, terbium, dysprosium, holmium, erbium, thulium, ytterbium, and lutetium; or said group of REEs comprises at least two REEs selected from the aforementioned list.

12. The process according to any one of claims 1 to 10, wherein said REE is selected from the group consisting of terbium, dysprosium, holmium, erbium, thulium, ytterbium, lutetium, scandium, yttrium; or said group of REEs comprises at least two REEs selected from the aforementioned list.

13. The process according to any one of claims 1 to 12, wherein said organism is from genus *Cupriavidus,* such as *Cupriavidus metallidurans,* or said organism is *Citrobacter* sp.

14. Use of biomass selected from the following organism classes: Eubacteria, Archaea, Algae, and Fungi for isolating a REE or group of REEs from a particulate material or a solution or dispersion containing said REE or said group of REEs.

15. A method of testing a sample microorganism for its ability to bind a rare earth element or of screening a plurality of microorganisms for the ability of members of said plurality to bind a rare earth element, comprising the following steps:
(a) contacting a microorganism with a solution or dispersion containing a rare earth element (REE) or multiple REEs;
(b) incubating the mixture of step (a) for a predetermined period of time;
(c) separating microorganisms from the mixture obtained in step (b);
(d) analysing the separated microorganism for bound REE.
